# EUROPEAN PATENT SPECIFICATION

(11) **EP 3 546 008 B1**
(45) Date of publication and mention of the grant of the patent: **07.08.2024**
(21) Application number: 17872921.6
(22) Date of filing: 24.11.2017
(51) Int. Cl.: A61M 25/00, A61M 25/092, A61M 25/098

(54) **CATHETER AND METHOD FOR MANUFACTURING CATHETER**
KATHETER UND VERFAHREN ZUR HERSTELLUNG DAVON
CATHÉTER ET PROCÉDÉ DE FABRICATION D'UN CATHÉTER

(30) Priority: 25.11.2016 JP 2016229418
(43) Date of publication of application: 02.10.2019
(73) Proprietor: Sumitomo Bakelite Co., Ltd., Shinagawa-ku Tokyo 140-0002 (JP)
(72) Inventor: KANEMASA Kenichi, Akita-shi Akita 011-8510 (JP); HAYASHI Nobuaki, Akita-shi Akita 011-8510 (JP); YAMAGUCHI Kenjiro, Akita-shi Akita 011-8510 (JP)
(74) Representative: Ter Meer Steinmeister & Partner
(86) International application number: PCT/JP2017/042259
(87) International publication number: WO 2018/097258

(56) References cited:
- EP-A1- 2 937 110
- JP-A- 2002 272 850
- JP-A- 2011 083 594
- JP-A- 2014 171 635
- JP-A- 2014 188 213
- JP-A- 2015 147 080
- JP-A- 2016 013 344
- JP-B2- H0 230 691
- US-A1- 2003 135 198
- US-A1- 2011 160 702
- US-A1- 2011 214 914
- US-B2- 8 486 048

## Description

### Technical Field

The present disclosure relates to a catheter and a method for manufacturing the catheter.

Priority is claimed on Japanese Patent Application No. 2016-229418, filed on November 25, 2016.

### Background Art

There is proposed a catheter in which a distal portion of a catheter is capable of being bent by pulling an operating line. When this type of catheter is inserted into a body cavity such as a blood vessel, it is possible to select an insertion direction by bending the distal portion of the catheter at a diverge point of the body cavity.

PTL 1 discloses one example of the catheter. The catheter disclosed in PTL 1 is configured to include a tubular main body that is long; an operating part that bends a distal portion of the tubular main body; and a holding wire.

The tubular main body has a reinforcing wire layer formed by winding a reinforcing wire around a main lumen; a sub-tube that is disposed outside the reinforcing wire layer and defines a secondary lumen having a smaller diameter than the main lumen; an outer layer including the reinforcing wire layer and the sub-tube; and a second reinforced layer which is provided closer to an outer diameter side than an operating line, to protect the outer layer, and which is formed by braiding a second reinforcing wire in a mesh pattern.

The operating part bends the distal portion of the tubular main body by pulling the operating line which is movably inserted into the secondary lumen, and the tip end of which is connected to the distal portion of the tubular main body.

The holding wire is a wire which collectively winds the sub-tube and the reinforcing wire layer which are included in the outer layer.

A first maker and a second marker are provided in the distal portion of the tubular main body, and are ring-shaped members containing a radiopaque material such as platinum which does not allow X-rays to penetrate therethrough. The markers indicate the respective positions of a tip end part and a bent part of the catheter in use, and serve as reference points for an operation performed by an operator.

A tip end portion of the operating line is fixed to the first marker. A distal side part of the second marker is flexible compared to part of the second marker which is closer to a proximal side than the distal side part. For this reason, it is possible to bend part of the catheter which is closer to a distal side than the second marker, and to change an insertion direction of the catheter by pulling the operating line.

### Citation List

### Patent Literature

[PTL 1] Japanese Unexamined Patent Application, First Publication No. 2014-171635

### Summary of Invention

### Technical Problem

In recent years, as a catheter is used for a variety of applications in the medical field, a reduction in the diameter of the catheter is required to enable the catheter to reach even a microvasculature.

On the other hand, there are various limitations to reducing the diameter of the catheter. A reduction in the diameter of the catheter needs to be achieved without bendability being adversely affected during the operation of the catheter. That is, when the catheter is operated, it is necessary to enable a predetermined part of the catheter to be sharply bent, and it is required to reduce the diameter of the catheter while enabling such sharp bending.

A reduction in the diameter of the catheter needs to be achieved without ease of assembly of the catheter being adversely affected. That is, when the catheter is manufactured, it is required to reduce the diameter of the catheter while enabling an easy and accurate assembly of configuration components of the catheter.

The present disclosure is made in light of the problems, and provides a catheter in which a reduction in diameter is capable of being realized without bendability or ease of assembly of the catheter being adversely affected. In addition, the present disclosure provides a method for manufacturing the catheter.

### Solution to Problem

The present disclosure includes the following technical concepts.
(1) There is provided a catheter comprising: a reinforcing wire layer wound on a main lumen; a first marker having a ring shape, and a second marker having a ring shape and positioned closer to a proximal side than the first marker, each the first and second markers being provided around the reinforcing wire layer and containing a radio-opaque metallic material; and an outer layer including the reinforcing wire layer, the first marker, and the second marker, wherein the reinforcing wire layer is formed in a layer beneath the first marker and the second marker, wherein the catheter further comprises a sub-tube, the sub-tube defining a secondary lumen having a smaller diameter than the main lumen; and an operating line which is movably inserted into the secondary lumen, and a tip end of which is connected to the first marker, and wherein the sub-tube is disposed outside the first marker and the second marker, and wherein the region between the first marker and the second marker is a non-forming region in which the reinforcing wire layer is not formed.
(2) The catheter described in (1) further includes a holding wire which collectively winds the reinforcing wire layer, the first marker, and the second marker, wherein the first marker and the second marker are provided in a layer beneath the holding wire.
(3) In the catheter described in (2), the holding wire is a coil formed by winding a plurality of strands multiple turns.
(4) In the catheter described in any one of (1) to (3), the reinforcing wire layer is a mesh layer.
(5) In the catheter described in (4), the reinforcing wire layer is a plurality of mesh layer formed at the same layer level in front and back of the non-forming region.
(6) In the catheter described in any one of (1) to (5), the first marker has the same outer diameter as the second marker, and the first marker and the second marker are disposed at the same height in a radial direction of the catheter.
(7) In the catheter described in any one of (1) to (6), the outer layer is formed of one type of material.
(8) In the catheter described in any one of (1) to (7), the outer layer has a substantially uniform thickness.
(9) In the catheter described in any one of (2) to (8), a material of each of the reinforcing wire layer and the holding wire is a metallic material.
(10) In the catheter described in any one of (1) to (9), a material of the sub-tube is a thermoplastic polymer.
(11) In the catheter described in any one of (1) to (10), the holding wire is in contact with an outside surface of the sub-tube at a position located opposite to an axis of the main lumen.
(12) There is provided a method for manufacturing a catheter including forming a reinforcing wire layer in which a reinforcing wire layer is formed by winding a reinforcing wire on an inner layer defining a main lumen; fixing a marker in which each of a first marker and a second marker is fixed, each of which contains a radiopaque metallic material and has a ring shape, to a circumference of the reinforcing wire layer by caulking such that the second marker is positioned closer to a proximal side than the first marker; removing a reinforcing wire layer in which the reinforcing wire layer between the first marker and the second marker is removed; and forming an outer layer in which an outer layer is formed so as to include the reinforcing wire layer, the first marker, and the second marker.
(13) The method for manufacturing a catheter described in (12) further includes collective winding in which a holding wire is wound collectively with the reinforcing wire layer, the first marker, and the second marker after removing the reinforcing wire layer.
(14) The method for manufacturing a catheter described in (12) or (13) further includes disposing a sub-tube in which a sub-tube is disposed, so that the sub-tube defines a secondary lumen having a smaller diameter than the main lumen, at a position outside the first marker and the second marker after removing the reinforcing wire layer; inserting an operating line in which an operating line is movably inserted into the secondary lumen after disposing the sub-tube; and connecting an operating line in which a tip end of the operating line is connected to the first marker after inserting the operating line.
(15) In the method for manufacturing a catheter described in any one of (12) to (14), the outer layer is formed of one type of material.
(16) In the method for manufacturing a catheter described in any one of (12) to (14), the outer layer has a substantially uniform thickness.
(17) In the method for manufacturing a catheter described in any one of (13) to (16), a material of each of the reinforcing wire layer and the holding wire is a metallic material.
(18) In the method for manufacturing a catheter described in any one of (14) to (17), a material of the sub-tube is a thermoplastic polymer.
(19) In the catheter described in any one of (14) to (18), the holding wire is in contact with an outside surface of the sub-tube at a position opposite to an axis of the main lumen.

### Advantageous Effects of Invention

In the catheter and the method for manufacturing the catheter, it is possible to reduce the diameter of the catheter without adversely affecting bendability or ease of assembly of the catheter.

### Brief Description of Drawings

FIG. 1 is an entire side view of a catheter of an embodiment of the present disclosure.
FIG. 2 is a longitudinal cross-sectional view of a tip end portion of the catheter.
FIG. 3 is a cross-sectional view taken along A-A line in FIG. 2.
FIG. 4 is a cross-sectional view taken along B-B line in FIG. 2.
FIG. 5 is a cross-sectional view taken along C-C line in FIG. 2.
FIG. 6 is an entire side view of the catheter illustrating a state where a wheel operating part is operated in one direction.
FIG. 7 is an entire side view of the catheter illustrating a state where the wheel operating part is operated in the other direction.
FIG. 8 is a longitudinal cross-sectional view illustrating a state where a reinforcing wire layer is wound on an inner layer.
FIG. 9 is a longitudinal cross-sectional view illustrating a state where each of a first marker, a second marker, and a third marker is fixed in a layer above the reinforcing wire layer.
FIG. 10 is a longitudinal cross-sectional view illustrating a state where the reinforcing wire layer in a region between the first marker and the second marker is removed from the configuration illustrated in FIG. 9.
FIG. 11 is a longitudinal cross-sectional view illustrating a state where sub-tubes are disposed which are added to the configuration illustrated in FIG. 10.
FIG. 12 is a longitudinal cross-sectional view illustrating a state where a holding wire is wound which is added to the configuration illustrated in FIG. 11.
FIG. 13 is a longitudinal cross-sectional view illustrating a state where an outer layer is formed which is added to the configuration illustrated in FIG. 12.
FIG. 14 is a longitudinal cross-sectional view illustrating a state where part of the outer layer located on a distal side is removed from the configuration illustrated in FIG. 13, a secondary core is withdrawn from each of the sub-tubes, an operating line is inserted into each of the sub-tubes, and an end portion of the operating line is fixed to the first marker.
FIG. 15 is a longitudinal cross-sectional view illustrating a state where an outer layer is formed again in a removed part of the outer layer located on the distal side, which is added to the configuration illustrated in FIG. 14.

### Description of Embodiments

Hereinbelow, an embodiment of the present disclosure will be described with reference to the drawings. In all the drawings, the same reference signs will be assigned to the same configuration elements, and duplicated descriptions will be appropriately omitted.

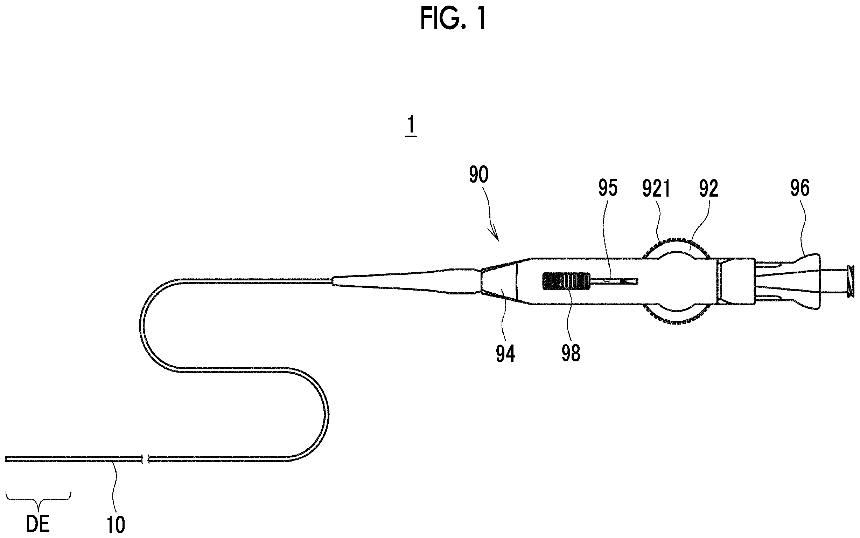

FIG. 1 is an entire side view of a catheter of the embodiment of the present disclosure.

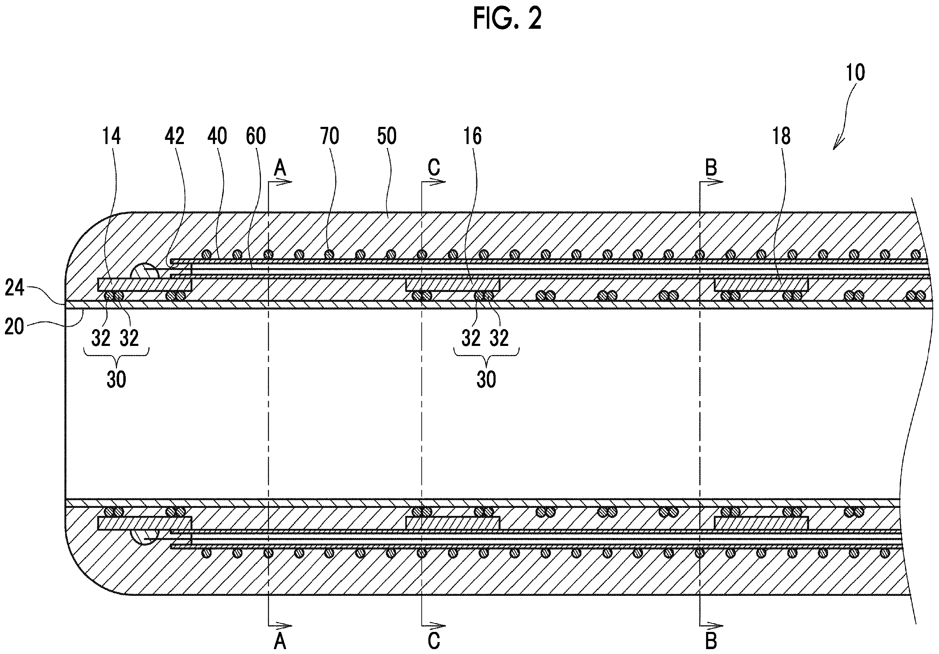

FIG. 2 is a longitudinal cross-sectional view of a tip end portion of the catheter.

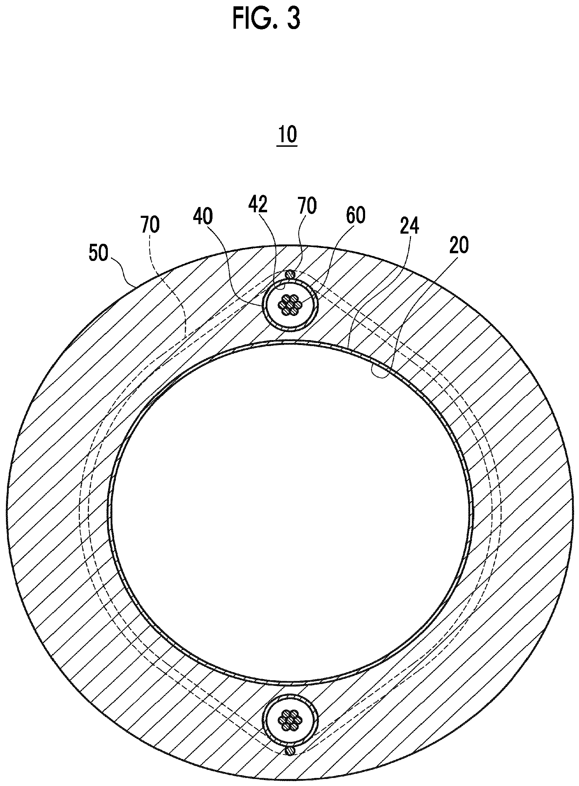

FIG. 3 is a cross-sectional view taken along A-A line in FIG. 2.

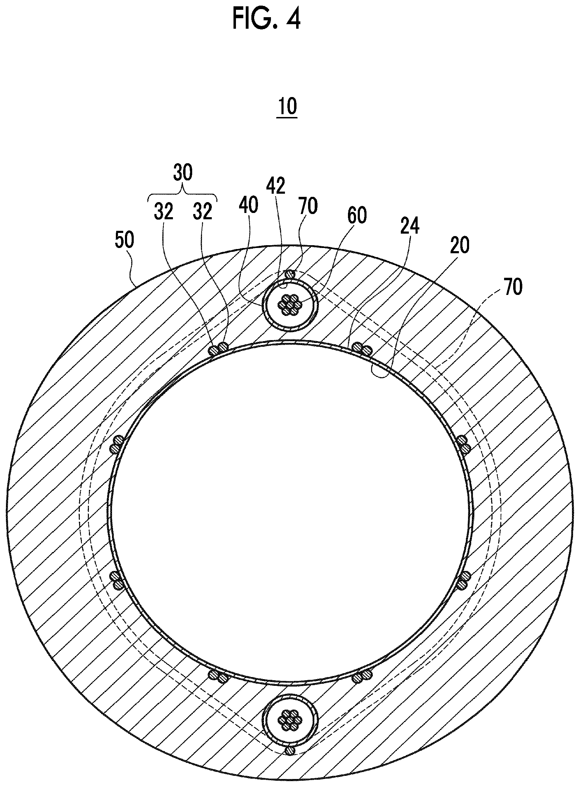

FIG. 4 is a cross-sectional view taken along B-B line in FIG. 2.

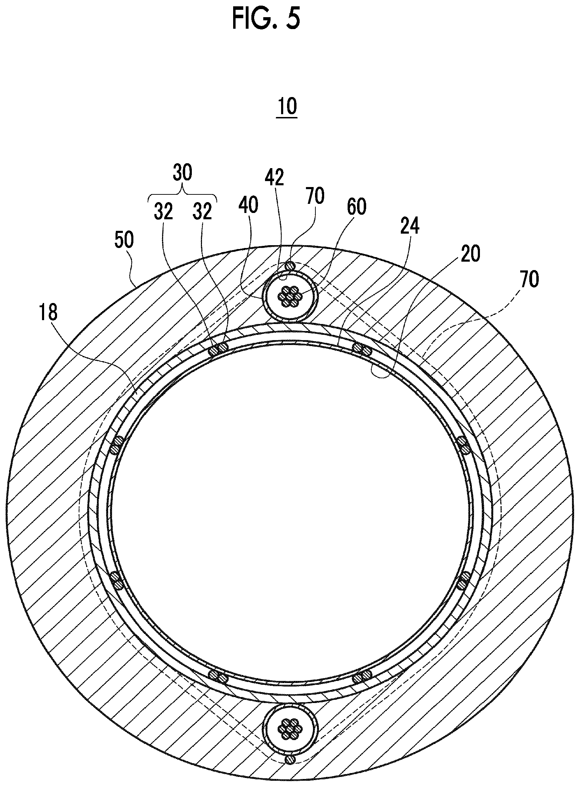

FIG. 5 is a cross-sectional view taken along C-C line in FIG. 2.

As illustrated in FIG. 2, a catheter 1 of the embodiment is provided with a reinforcing wire layer wound on a main lumen; a first marker having a ring shape and a second marker having a ring shape and positioned closer to a proximal side than the first marker, each of the first and second markers being provided around the reinforcing wire layer and containing a radiopaque metallic material; and an outer layer including the reinforcing wire layer, the first marker, and the second marker. In the catheter 1 of the embodiment, the reinforcing wire layer is formed in a layer beneath the first marker and the second marker, and the region between the first marker and the second marker is a non-forming region in which the reinforcing wire layer is not formed.

A holding wire 70 is provided inside a tubular main body 10, and collectively winds the reinforcing wire layer 30, the first marker 14, and the second marker 16. Each of the first marker 14 and the second marker 16 is provided in a layer beneath the holding wire 70.

The catheter 1 of the embodiment further includes a sub-tube 40 which is disposed outside the first marker 14 and the second marker 16, and defines a secondary lumen 42 having a smaller diameter than a main lumen 20; and an operating line 60 which is slidably inserted into the secondary lumen 42, and the tip end of which is connected to the first marker 14.

The catheter 1 of the embodiment includes the sub-tube 40 and the operating line 60; however, in the present disclosure, the sub-tube 40 and the operating line 60 are not necessarily required as configuration elements, and the catheter 1 may not include the sub-tube 40 and the operating line 60.

The catheter 1 further includes a third marker 18 inside the tubular main body 10. The third marker 18 is a ring-shaped member which is provided around the reinforcing wire layer 30, and which is made of a radiopaque metallic material. The third marker 18 is positioned closer to the proximal side than the second marker 16. The holding wire 70 is wound around the third marker 18 together with the first marker 14 and the second marker 16. The third marker 18 is provided in the layer beneath the holding wire 70. In the present disclosure, the third marker 18 is not necessarily required as a configuration element, and is capable of being omitted.

Hereinbelow, the embodiment will be described in detail. The catheter 1 of the embodiment is an intravascular catheter which is used in such a way that the tubular main body 10 is inserted into a blood vessel

The tubular main body 10 is referred to as a sheath, and is a long hollow tubular member in which the main lumen 20 is formed as a through hole.

The tubular main body 10 has a stack structure. The tubular main body 10 is configured such that the main lumen 20 is disposed at the center of the tubular main body 10 and an inner layer (main tube) 24 and an outer layer 50 are stacked on top of each other sequentially from an inner diameter side of the tubular main body 10. A hydrophilic layer (not illustrated) is formed on a surface of the outer layer 50. Each of the inner layer 24 and the outer layer 50 is formed of a flexible resin material, and has a circular tubular shape and a substantially uniform thickness.

The inner layer 24 is an innermost layer of the tubular main body 10, and the main lumen 20 is defined by an inner wall surface of the inner layer 24. The cross-sectional shape of the main lumen 20 is not limited to a specific shape, and is a circular shape in the embodiment. If the vertical cross section of the main lumen 20 is a circular shape, the diameter of the main lumen 20 may be uniform along a longitudinal direction of the tubular main body 10, or may differ depending on positions in the longitudinal direction. The main lumen 20 is capable of having a tapered shape in which the diameter of the main lumen 20 continuously increases from a tip end to a base end of the main lumen 20 in the entire region or part of the region along the length of the tubular main body 10.

The inner layer preferably has a substantially uniform thickness from the viewpoint of enabling an easy and accurate assembly

The inner layer may be formed of the same type of material or different types of materials. The inner layer is preferably formed of the same type of material from the viewpoint of enabling an easy and accurate assembly.

For example, it is possible to use a fluorine thermoplastic polymer resin as the material of the inner layer 24. Specifically, examples of the fluorine thermoplastic polymer material are capable of including a polytetrafluoroethylene (PTFE) resin, a polyvinylidene fluoride (PVDF) resin, and a perfluoroalkoxy (PFA) resin. If the inner layer 24 is formed of a fluorine polymer material, a drug solution or the like is well delivered when being supplied via the main lumen 20. A sliding resistance when a guide wire or the like is being inserted into the main lumen 20 is reduced.

A reinforcing wire layer 30 is a protective layer which is provided closer to the inner diameter side in the tubular main body 10 than the operating line 60, and which protects the inner layer 24. Because the reinforcing wire layer 30 is present closer to the inner diameter side than the operating line 60, the operating line 60 is capable of being prevented from penetrating through the layers from the outer layer 50 to the inner layer 24, and being exposed to the main lumen 20.

The reinforcing wire layer 30 is formed by winding a reinforcing wire 32. As the material of the reinforcing wire 32, in addition to a metallic material such as tungsten (W), a stainless steel (SUS), a nickel-titanium alloy, a steel, titanium, copper, a titanium alloy or a copper alloy, it is possible to use a resin material such as polyimide (PI), polyamideimide (PAI) or polyethylene terephthalate (PET) which has a shear strength greater than that of each of the inner layer 24 and the outer layer 50. A metallic material is preferably used as the material of the reinforcing wire layer 32. In the embodiment, a thin stainless steel wire is used as the material of the reinforcing wire 32.

The reinforcing wire layer 30 is a mesh layer. Because the layer beneath each of a first marker 14, a second marker 16, and a third marker 18 is the reinforcing wire layer 30 that is formed as a mesh layer resistant to a mechanical deformation, each of the first marker 14, the second marker 16, and the third marker 18 is capable of being fixed to the reinforcing wire layer 30 via caulking.

The number of turns of the reinforcing wire 32 or the number of meshes is not limited to a specific number of turns or meshes. The number of meshes of the reinforcing wire layer 30 refers to the number of crossings (the number of openings) per unit length (one inch) in an extending direction of the reinforcing wire.

The reinforcing wire 32 is obliquely wound on the inner layer 24. A pitch angle of the reinforcing wire 32 refers to an angle formed by the extending direction of the reinforcing wire 32 and a radial direction of the inner layer 24. If the reinforcing wire 32 is wound with a tight pitch, the pitch angle becomes a small angle. On the other hands, if the reinforcing wire 32 is wound at a shallow angle along an axis of the tubular main body 10, the pitch angle becomes a large angle close to 90 degrees. In the embodiment, the pitch angle of the reinforcing wire 32 is not limited to a specific pitch angle, and is capable of being greater than or equal to 30 degrees, preferably, greater than or equal to 45 degrees and less than or equal to 75 degrees.

The number of turns of the reinforcing wire 32 of the reinforcing wire layer 30 is not limited to a specific number of turns. The embodiment illustrates that the reinforcing wire layer 30 is formed of 16 turns of the reinforcing wire 32.

A distal portion DE of the tubular main body 10 is provided with the first marker 14, and the second marker 16 that is positioned closer to the proximal side than the first marker 14. The first marker 14 and the second marker 16 are ring-shaped members containing a radiopaque metallic material such as platinum which does not allow X-rays to penetrate therethrough. Because the positions of two markers, that is, the first marker 14 and the second marker 16 are used as indexes, the position of a tip end of the tubular main body 10 inside a body cavity (blood vessel) is capable of being visualized by radiation beam (X-ray) observation. Therefore, an operator is capable of easily determining an optimal timing for bending the catheter 1.

The third marker 18 is provided closer to the proximal side than the second marker 16. The third marker 18 also is an index for the operation of the catheter 1. The embodiment illustrates that a side surface (positioned on a distal side) of the third marker 18 is positioned apart by 30 mm from the tip end of the tubular main body 10.

If the catheter 1 is used to introduce a detachable coil into the body of a subject, an operator is capable of understanding the cut position of the detachable coil inside the main lumen 20 of the catheter 1 by using the third marker 18 as a reference point. The detachable coil refers to a coil that indwells inside the body of the subject to embolize an aneurysm or thrombus of the subject. It is possible to cut the detachable coil at a predetermined cut position by energizing the detachable coil inside the main lumen 20.

In the embodiment, all of the first marker 14, the second marker 16, and the third marker 18 are members which contain the same material and have the same shape.

The first marker 14 has the same outer diameter as the second marker 16, and the first marker 14 and the second marker 16 are disposed at the same height in the radial direction of the catheter 1.

Because the first marker 14 and the second marker 16 are disposed at the same height, the sub-tube 40 is capable of being straightly disposed in a layer above the first marker 14 and the second marker 16. For this reason, when the catheter 1 is manufacture, a corner portion caused by the bending of the sub-tube 40 is not formed in a region inside the secondary lumen 42, which corresponds to the distal portion DE of the sub-tube 40. Therefore, the sub-tube 40 is capable of being effectively prevented from being worn out and damaged due to the operating line 60 rubbing against the corner portion when the operating part 90 is operated.

The third marker 18 is a member which contains the same material and has the same shape as the material and the shape of the first marker 14 and the second marker 16.

Because the first marker 14, the second marker 16, and the third marker 18 are made of the same member, it is possible to prepare the first marker 14, the second marker 16, and the third marker 18 using a common mold, and compared to when the first marker 14, the second marker 16, and the third marker 18 are made of members having different shapes, respectively, it is possible to reduce manufacturing costs.

The reinforcing wire layer 30 is formed in the layer beneath each of the first marker 14, the second marker 16, and the third marker 18, and is not formed in a region between the first marker 14 and the second marker 16. The region is referred to a non-forming region in which the reinforcing wire layer 30 is not formed.

There is a discontinuity in the flexural rigidity of the tubular main body 10 between the region in which the reinforcing wire layer 30 is not formed and a region in which the reinforcing wire layer 30 is formed. The flexural rigidity in the non-forming region in which the reinforcing wire layer 30 is not formed is less than the flexural rigidity in the region in which the reinforcing wire layer 30 is formed. For this reason, when the operating line 60 is pulled, the tubular main body 10 is capable of being sharply bent in the non-forming region in which the reinforcing wire layer 30 is not formed.

The reinforcing wire layer 30 is a plurality of mesh layer formed at the same layer level in front and back of the non-forming region in which the reinforcing wire layer 30 is not reformed.

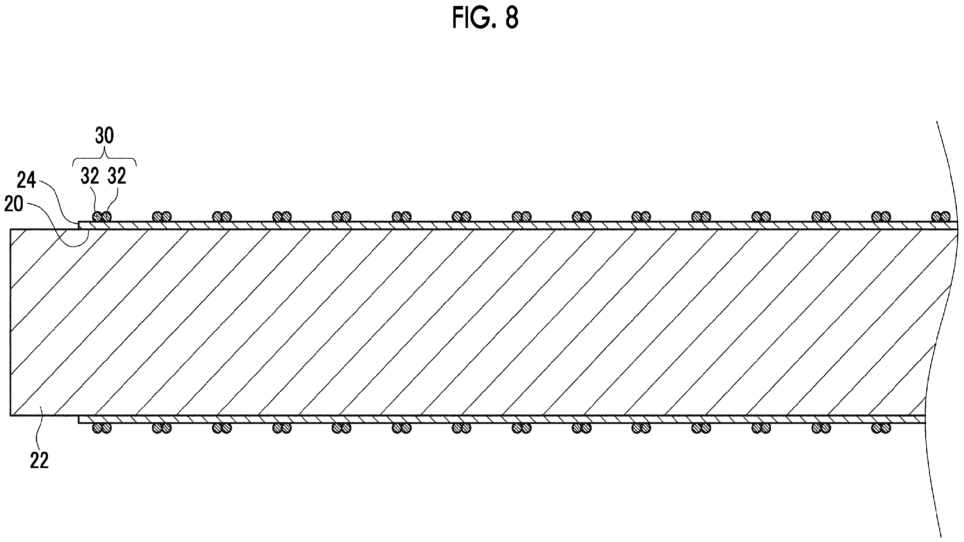

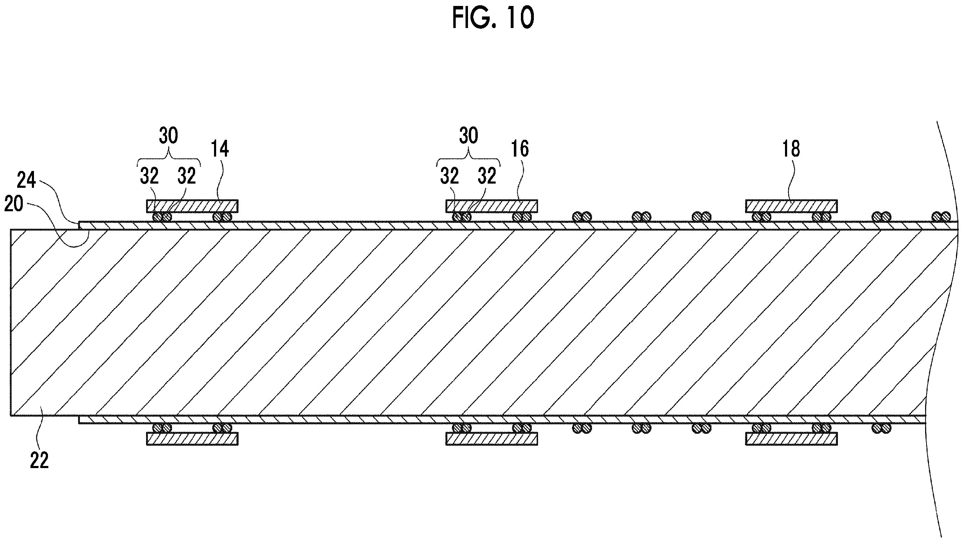

Initially, as illustrated in FIG. 8, the reinforcing wire 32 is wound in a mesh pattern on the entirety of the inner layer 24, and then as illustrated in FIG. 10, each of the first marker 14, the second marker 16, and the third marker 18 is fixed to the reinforcing wire layer 30 via caulking, and part of the reinforcing wire 32 which corresponds to the non-forming region is removed, and therefore, the reinforcing wire layer 30 is formed, which will be described later in detail. For this reason, the reinforcing wire layers 30 become mesh layers formed at the same layer level in front and back of the non-forming region in which the reinforcing wire layer 30 is not reformed. It is possible to easily form the reinforcing wire layers 30 which are mesh layers formed at the same layer level in front and back of the non-forming region, and it is possible to improve ease of manufacturing of the catheter 1.

The reinforcing wire layer 30 which is a mesh layer is provided in the layer beneath each of the first marker 14, the second marker 16, and the third marker 18.

Each of the first marker 14, the second marker 16, and the third marker 18 is fixed to the reinforcing wire layer 30 via caulking. If the reinforcing wire layer 30 is not provided, the first marker 14, the second marker 16, and the third marker 18 are directly fixed to the inner layer 24 via caulking. On the other hands, because the inner layer 24 is made of a flexible material, the inner layer 24 is easily deformed, it is difficult to directly fix the first marker 14, the second marker 16, and the third marker 18 to the inner layer 24 via caulking, and if the inner layer 24 is deformed, the first marker 14, the second marker 16, and the third marker 18 easily detach from the inner layer 24 even though fixed to the inner layer 24.

In the embodiment, because the reinforcing wire layer 30 is a mesh layer that is more resistant to a mechanical deformation than the inner layer 24, and is provided in the layer beneath each of the first marker 14, the second marker 16, and the third marker 18, each of the first marker 14, the second marker 16, and the third marker 18 is capable of being fixed to the reinforcing wire layer 30 via caulking, and is also capable of being prevented from detaching from the reinforcing wire layer 30 after fixed to the reinforcing wire layer 30.

The sub-tube 40 is a hollow tubular member that defines a secondary lumen. The sub-tube 40 is embedded inside of the outer layer 50. The sub-tube 40 is capable of being formed of a thermoplastic polymer material. An example of the thermoplastic polymer material is a low frictional resin material such as polytetrafluoroethylene (PTFE), polyetheretherketone (PEEK), or a tetrafluoroethylene/hexafluoropropylene copolymer (FEP). The sub-tube 40 is formed of a material having a flexural rigidity and a tensile modulus greater than the flexural rigidity and the tensile modulus of the outer layer 50.

An outer surface of the sub-tube 40 is subject to an etching treatment such as a metal sodium treatment or a plasma treatment. Therefore, adhesion between the sub-tube 40 and the outer layer improves.

As illustrated in FIGS. 3 to 5, two sub-tubes 40 are disposed in the surroundings of the reinforcing wire layers 30 while being 180 degrees opposite to each other. The operating lines 60 are inserted into two sub-tubes 40, respectively. Two sub-tubes 40 are parallel to an axial direction of the tubular main body 10.

As illustrated in FIGS. 3 to 5, two sub-tubes 40 are disposed on the same circumference to surround the main lumen 20. Instead of two sub-tubes 40 being provided as in the embodiment, three or four sub-tubes 40 may be disposed in the surroundings of the main lumen 20 while being equally spaced from each other. In this case, the operating lines 60 may be disposed for all of the sub-tubes 40, respectively, or the operating lines 60 may be disposed for part of the sub-tubes 40.

The operating lines 60 are loosely and slidably inserted into the sub-tubes 40, respectively. A tip end portion of each of the operating lines 60 is fixed to the distal portion DE of the tubular main body 10. If the operating line 60 is pulled toward a base end side, because tubular main body 10 is subject to a tensile force toward a position offset relative to the axis of the tubular main body 10, the tubular main body 10 is bent. Because the operating line 60 of the embodiment is extremely thin and highly flexible, even though the operating line 60 is pushed toward the distal side, a pushing force is not actually applied to the distal portion DE of the tubular main body 10.

The operating line 60 may be formed of a single wire, or may be a stranded wire formed by twisting together a plurality of thin wires. The number of the thin wires forming one stranded wire of the operating line 60 is not limited to a specific number, and preferably is three or more. A preferred example of the number of the thin wires is seven or three. The operating line 60 of the embodiment is a stranded wire formed by twisting together seven strands. More specifically, seven strands are integrally twisted together by spirally winding six strands (peripheral strands) on one strand (center strand) located at the center of the strands. Six peripheral strands are disposed at the vertices of a hexagon having the center strand at the center of the hexagon, respectively.

As the material of the operating line 60, it is possible to use a metallic wire such as a low carbon steel wire (piano wire), a stainless steel (SUS) wire, a corrosion-resistant coated steel wire, a titanium wire, a titanium alloy wire, or a tungsten wire. As the material of the operating line 60, in addition to the foregoing materials, it is possible to use a polymer fiber such as polyvinylidene fluoride (PVDF), high-density polyethylene (HDPE), poly (paraphenylene benzobisoxazole) (PBO), polyetheretherketone (PEEK), polyphenylene sulfide (PPS), polybutylene terephthalate (PBT), polyimide (PI), polytetrafluoroethylene (PTFE), or a boron fiber.

The tip end portion of the operating lines 60 is fixed to an outer circumferential part of the first marker 14. A method for fixing the operating line 60 to the first marker 14 is not limited to a specific method. Examples of the fixing method are capable of including soldering, heat fusion, bonding via an adhesive, and mechanical latching between the operating line 60 and the first marker 14. In the embodiment, the operating line 60 is fixed to the outer circumferential part of the first marker 14 via soldering.

The holding wire 70 collectively winds the sub-tubes 40, the first marker 14, the second marker 16, the third marker 18, and the reinforcing wire layers 30. The holding wire 70 is formed around the sub-tubes 40 by coil winding or mesh braiding.

The holding wire 70 of the embodiment is a coil, more specifically, a coil (multiple-turn coil) formed by winding a plurality of strands multiple turns.

The holding wire 70 is spirally wound to surround the outside of a pair of the sub-tubes 40 that are disposed in the surroundings of the main lumen 20 while facing each other. In the embodiment, the winding shape of the holding wire 70 is a substantially elliptical shape or a substantially rhombus shape, the major axis direction of which is the same as an alignment direction of the sub-tube 40. In FIGS. 3-5, the holding wire 70 having a substantially rhombus shape as the winding shape is illustrated by the dotted line. The holding wire 70 is in contact with a circumferential surface, specifically, an outside surface (located opposite to the axis of the main lumen 20) of each of the sub-tubes 40. The substantially rhombus shape implies a shape in which a first diagonal line is longer than a second diagonal line, and the first diagonal line is substantially perpendicular to the second diagonal line. The substantially rhombus shape referred to herein includes a kite shape, or a flat polygonal shape such as a flat hexagonal shape or flat octagonal shape in addition to a rhombus shape. The substantially elliptical shape includes an eccentric circular shape such as an egg shape in addition to an elliptical shape or oval shape.

The embodiment exemplifies the configuration in which the main lumen 20 has a circular shape and is disposed at the center of the tubular main body 10, and two sub-tubes 40 are disposed in the surroundings of the main lumen 20 while being 180 degrees opposite to each other. Three or more (N number of) sub-tubes 40 may be distributed in the surroundings of the main lumen 20 while being equally disposed from each other. In this case, the winding shape of the holding wire 70 may be an N-polygonal shape with rounded corners which has the sub-tubes 40 at the corners, respectively.

In a forming region in which the reinforcing wire layers 30 are formed, the holding wire 70 is in contact with an outer surface of the reinforcing wire layer 30 on both sides or a single side in a minor axis direction perpendicular to the major axis direction of the winding shape.

An inside surface of each of the sub-tubes 40 is in contact with an outer surface of each of the first marker 14, the second marker 16, and the third marker 18 (refer to FIG. 2). The holding wire 70 is spirally wound on the pair of sub-tubes 40 while being in contact with the outside surface of each of the sub-tubes 40. The holding wire 70 is wound on the sub-tubes 40 over the entire length of the sub-tubes 40 in the longitudinal direction of the tubular main body 10.

Therefore, the holding wire 70 collectively winds the sub-tubes 40, the first marker 14, the second marker 16, the third marker 18, and the reinforcing wire layers 30 that are the layers beneath the first marker 14, the second marker 16, and the third marker 18, such that the sub-tubes 40, the first marker 14, the second marker 16, the third marker 18, and the reinforcing wire layers 30 are in close contact with each other without being loosely joined together. For this reason, even after a process of forming the outer layer 50, the sub-tubes 40 are capable of highly accurately keeping parallel to the first marker 14, the second marker 16, and the third marker 18, or parallel to the reinforcing wire layers 30, and the position of each of the sub-tubes 40 is capable of being prevented from being offset. The position of each of the sub-tubes 40 is more effectively prevented from being offset because a tightening force increases by virtue of the holding wire 70 being a multiple-turn coil.

Any one of the metallic materials or the resin materials capable of being used as the material of the reinforcing wire 32 is capable of being used as the material of the holding wire 70. A metallic material is preferably used as the material of the holding wire 70. In the embodiment, the holding wire 70 is formed to contain a different type of material which is not contained in the reinforcing wire 32. The ductility of the holding wire 70 is preferably greater than the ductility of the reinforcing wire 32. Specifically, the holding wire 70 is capable of being made of a soft austenitic stainless steel (W1 or W2) that is an annealed material, copper, or a copper alloy, and the reinforcing wire 32 is capable of being made of a tungsten or stainless spring steel.

Because a highly ductile material is used as the material of the holding wire 70, when the holding wire 70 is formed around the sub-tubes 40 by coil winding or mesh braiding (coil winding in the embodiment), the holding wire 70 plastically stretches and deforms without the winding of the holding wire 70 being loose, thereby fixing the sub-tubes 40.

A proximal end of the reinforcing wire layer 30 is positioned inside a proximal end of the tubular main body 10, that is, inside the operating part 90.

A distal end of the inner layer 24 may reach a distal end of the tubular main body 10, or may end at a position that is away from the distal end toward the base end side. The position where the inner layer 24 ends may be inside a region in which the first marker 14 is provided.

The outer layer 50 is a part having a circular tubular shape which mainly forms a thickness of the tubular main body 10. The reinforcing wire layer 30, the first marker 14, the second marker 16, the third marker 18, the sub-tube 40, and the holding wire 70 are provided inside of the outer layer 50 sequentially from the inner diameter side. The reinforcing wire layers 30, the first marker 14, the second marker 16, the third marker 18, and the holding wire 70 are disposed coaxially with the tubular main body 10.

The outer layer preferably has a substantially uniform thickness from the viewpoint of enabling an easy and accurate assembly.

The outer layer may be formed of the same type of material or different types of materials. The outer layer is preferably formed of the same type of material from the viewpoint of enabling an easy and accurate assembly.

It is possible to use a thermoplastic polymer material as the material of the outer layer 50. Examples of the thermoplastic polymer material are capable of including nylon elastomers such as polyimide (PI), polyamideimide (PAI), polyethylene terephthalate (PET), polyethylene (PE), polyamide (PA), a polyamide elastomer (PAE), polyether block amide (PEBA), and including polyurethane (PU), an ethylene-vinyl acetate (EVA) resin, polyvinyl chloride (PVC), and polypropylene (PP).

Inorganic fillers may be mixed in the outer layer 50. A contrast agent such as barium sulfate or bismuth subcarbonate is capable of being an example of the inorganic filler. The mixing of the contrast agent in the outer layer 50 is capable of improving the X-ray contrast properties of the tubular main body 10 inside a body cavity.

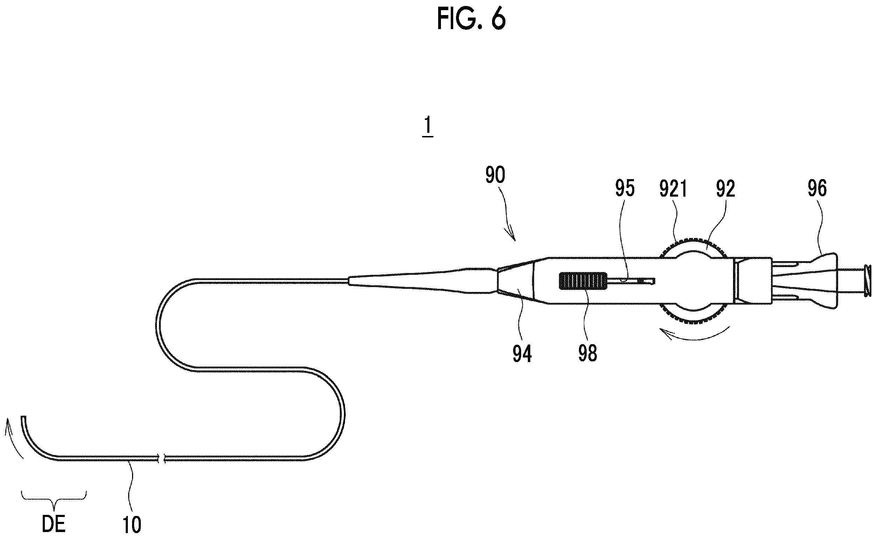

FIG. 6 is an entire side view of the catheter illustrating a state where a wheel operating part is operated in one direction.

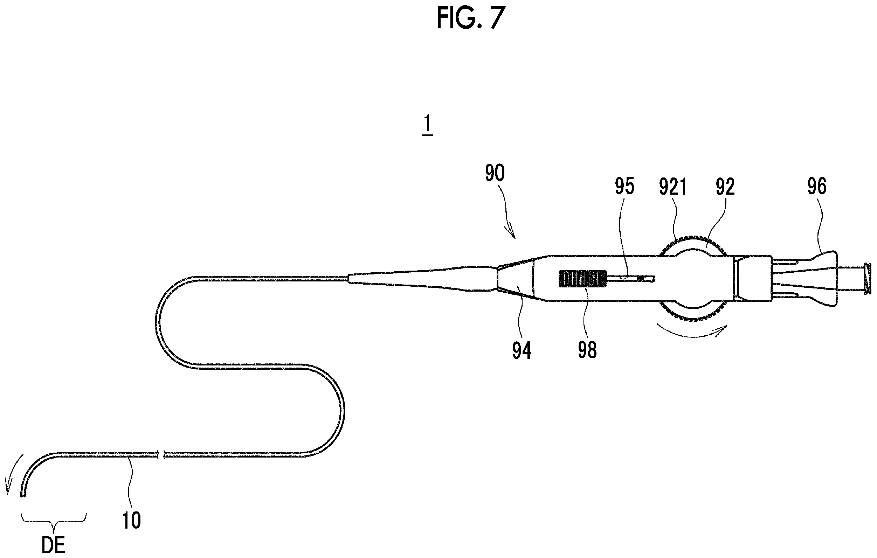

FIG. 7 is an entire side view of the catheter illustrating a state where the wheel operating part is operated in the other direction.

The catheter 1 has the operating part 90 provided in a base end portion of the tubular main body 10. Along with the operating lines 60 (refer to FIG. 2), the operating part 90 forms an operating mechanism for bending the distal portion DE of the tubular main body 10.

The operating part 90 has a main body case 94 which a user grips by the hand, and a wheel operating part 92 which is provided rotatably relative to the main body case 94. The base end portion of the tubular main body 10 is introduced into the main body case 94.

The operating part 90 includes a hub 96 provided to communicate with the main lumen 20 of the tubular main body 10. A syringe (not illustrated) is mounted in the hub 96. The hub 96 is provided in a rear end portion of the main body case 94, and the syringe is mounted in the hub 96 from a rear side of the hub 96. A user is capable of supplying a drug solution or the like into a body cavity of a subject via the main lumen 20 by injecting the drug solution or the like into the hub 96 via the syringe. Examples of the drug solution are capable of including a medical device such as a detachable coil or beads (spherical embolic substance) in addition to drug solutions such as a contrast agent, anticancer liquid, a physiological saline solution, and n-butyl-2-cianoacrylate (NBCA) used as an instant adhesive.

The operating lines 60 and the sub-tubes 40 diverge from the tubular main body 10 in a front end portion of the inner space of the main body case 94, respectively. A base end portion of each of the operating lines 60, which leads out of each of two sub-tubes 40, is directly or indirectly connected to the wheel operating part 92.

If the wheel operating part 92 rotates in any direction, because one of two operating lines 60 is pulled toward the base end side, one operating line 60 is capable of being subject to a tensile force, and the other operating line 60 is capable of becoming loose. Therefore, the pulled operating line 60 bends the distal portion DE of the tubular main body 10.

Specifically, as illustrated in FIG. 6, if the wheel operating part 92 rotates in one direction (clockwise direction), one operating line 60 is pulled toward the base end side. A tensile force is applied to the distal portion DE of the tubular main body 10 via one operating line 60. Therefore, the distal portion DE of the tubular main body 10 is bent relative to a datum line, that is, the axis of the tubular main body 10 toward the sub-tube 40 into which one operating line 60 is inserted.

As illustrated in FIG. 7, if the wheel operating part 92 rotates a rotary shaft of the wheel operating part 92 in the other direction (counterclockwise direction), the other operating line 60 is pulled toward the base end side. A tensile force is applied to the distal portion DE of the tubular main body 10 via the other operating line 60. Therefore, the distal portion DE of the tubular main body 10 is bent relative to the datum line, that is, the axis of the tubular main body 10 toward the sub-tube 40 into which the other operating line 60 is inserted.

If two operating lines 60 are selectively pulled by the operation of the wheel operating part 92 of the operating part 90, the distal portion DE of the tubular main body 10 is capable of being bent selectively in a first or second direction which is contained in the same plane.

A toothed engaging portion 921 is formed in a circumferential surface of the wheel operating part 92. In the embodiment, a knurled portion having wave-like vertical ridges is exemplified.

A recessed portion 95 is formed in the main body case 94, and is located at a position where the main body case 94 is in contact with the wheel operating part 92. The recessed portion 95 is provided with a slider 98 that slides to advance and retract from the wheel operating part 92. A protrusion (not illustrated) is formed in a tip end portion of the slider 98, which is a lower (back side of the drawing sheet of FIG. 1) portion of the slider 98 and is toward the wheel operating part 92.

The protrusion has a size less than an opening width of the toothed engaging portion (knurled portion having vertical ridges) 921 formed in the circumferential surface of the wheel operating part 92. For this reason, if the slider 98 slides to the wheel operating part 92, the protrusion latches onto the circumferential surface of the wheel operating part 92, and the rotation of the wheel operating part 92 is restricted. Therefore, the wheel operating part 92 is capable of being restricted from rotating in a state where the distal portion DE of the tubular main body 10 is bent, and the catheter 1 is capable of maintaining a bent state.

If the operating part 90 rotates around the axis of the tubular main body 10, the distal portion DE of the tubular main body 10 is capable of being subject to torque and rotating at a predetermined angle. Therefore, the direction of the distal portion DE of the catheter 1 is capable of being freely controlled by a combination of the operation of the wheel operating part 92 and the rotation of the entirety of the operating part 90 around the axis. If the magnitude of the rotary angle of the wheel operating part 92 is adjusted, the pulling length of the operating line 60 is adjusted to a predetermined length, and thus the bending angle of the distal portion DE of the catheter 1 is capable of being controlled. For this reason, the catheter 1 is capable of being pushed into and entering a body cavity such as blood vessels diverging at various angles.

In a catheter of the related art, bendability is endowed to a distal portion of a tubular main body by a difference in rigidity between a part in which one reinforcing wire layer is provided and a part in which two reinforcing wire layers are provided. In the present disclosure, the non-forming region is provided in which the reinforcing wire layer 30 is not formed, and bendability is endowed to the tubular main body 10 by a difference in rigidity between in the non-forming region and a peripheral region in which the reinforcing wire layer 30 is formed.

In the present invention, because bendability is endowed by a change in rigidity induced by providing the non-forming region in which the reinforcing wire layer 30 is not formed, it is possible to reduce the number of layers forming the tubular main body 10, and it is possible to realize a reduction in the diameter of the catheter 1 while securing good bendability of the catheter 1.

Because the reinforcing wire layer 30 is formed in the layer beneath each of the first marker 14, the second marker 16, and the third marker 18, as described above, it is possible to fix each of the first marker 14, the second marker 16, and third marker 18 to the reinforcing wire layer 30 via caulking, and it is possible to realize a reduction in the diameter of the catheter 1 while securing ease of assembly of the catheter 1.

### (Manufacturing Method)

Subsequently, a method for manufacturing the catheter 1 of the embodiment will be described with reference to FIGS. 8 to 15.

FIG. 8 is a longitudinal cross-sectional view illustrating a state where the reinforcing wire layer 30 is wound on the inner layer 24.

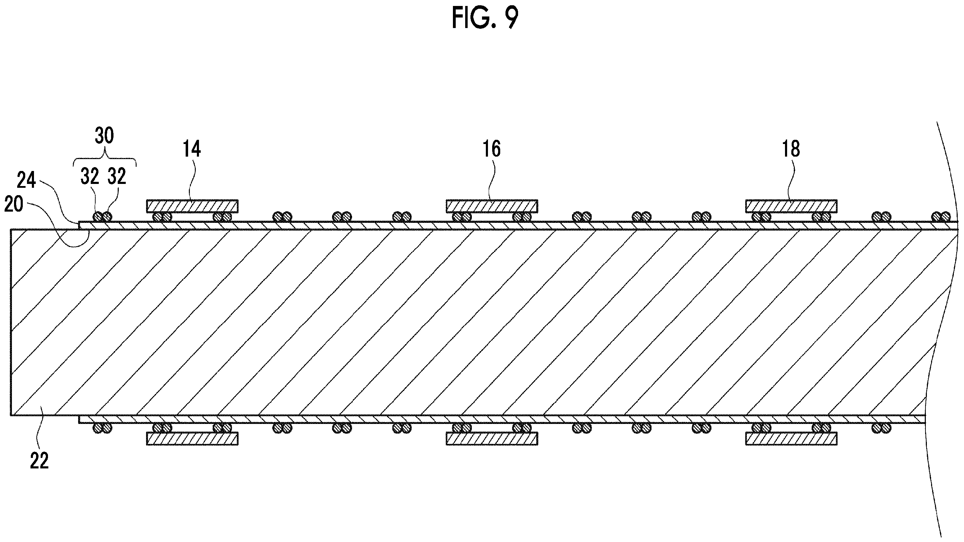

FIG. 9 is a longitudinal cross-sectional view illustrating a state where each of the first marker 14, the second marker 16, and the third marker 18 is fixed in a layer above the reinforcing wire layer 30.

FIG. 10 is a longitudinal cross-sectional view illustrating a state where the reinforcing wire layer 30 in a region between the first marker 14 and the second marker 16 is removed from the configuration illustrated in FIG. 9.

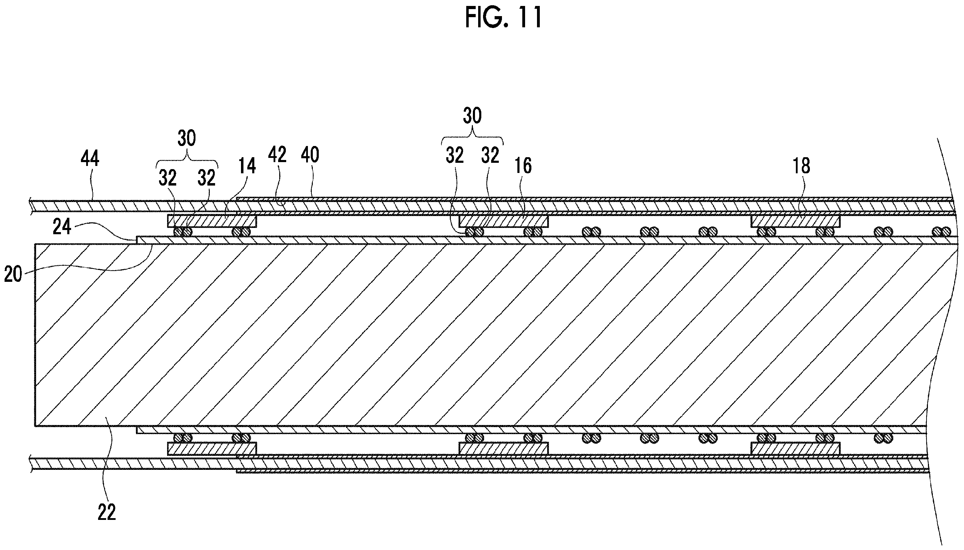

FIG. 11 is a longitudinal cross-sectional view illustrating a state where the sub-tubes 40 are disposed which are added to the configuration illustrated in FIG. 10.

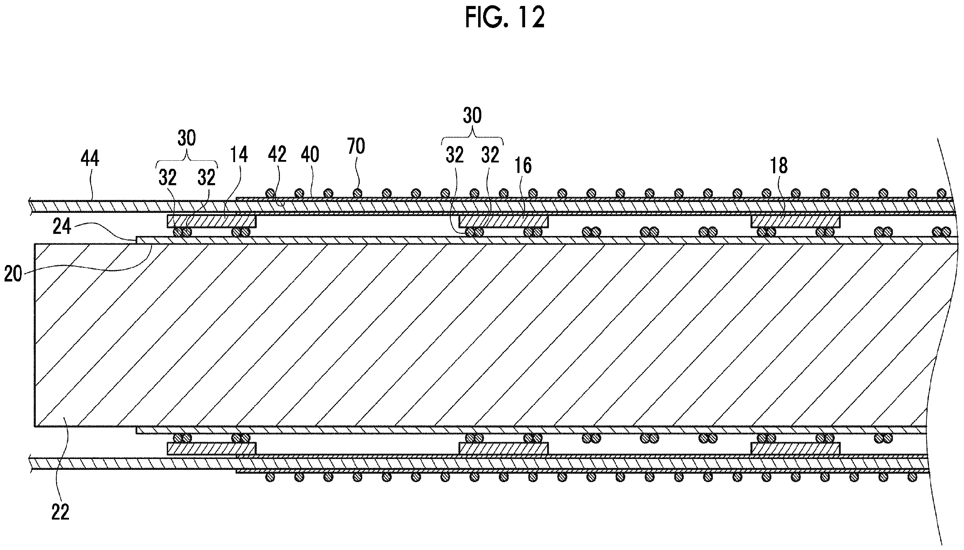

FIG. 12 is a longitudinal cross-sectional view illustrating a state where the holding wire 70 is wound which is added to the configuration illustrated in FIG. 11.

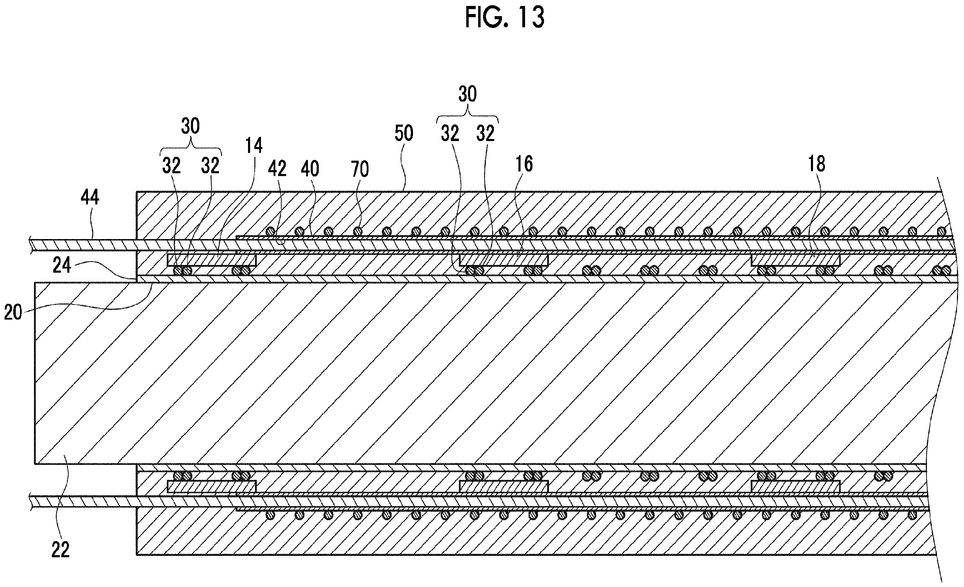

FIG. 13 is a longitudinal cross-sectional view illustrating a state where the outer layer 50 is formed which is added to the configuration illustrated in FIG. 12.

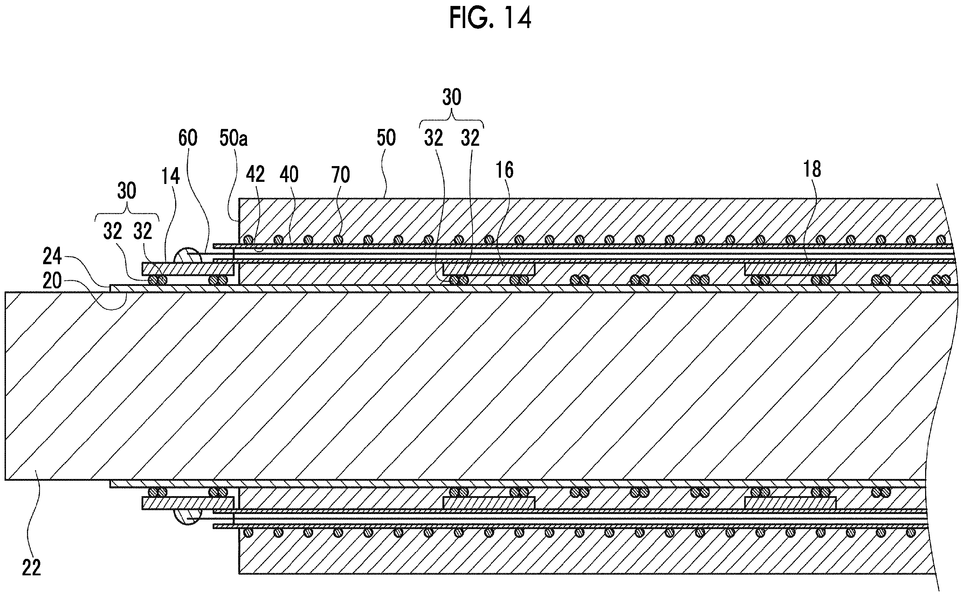

FIG. 14 is a longitudinal cross-sectional view illustrating a state where part of the outer layer 50 located on the distal side is removed from the configuration illustrated in FIG. 13, a secondary core 44 is withdrawn from each of the sub-tubes 40, the operating line 60 is inserted into each of the sub-tubes 40, and an end portion of the operating line 60 is fixed to the first marker 14.

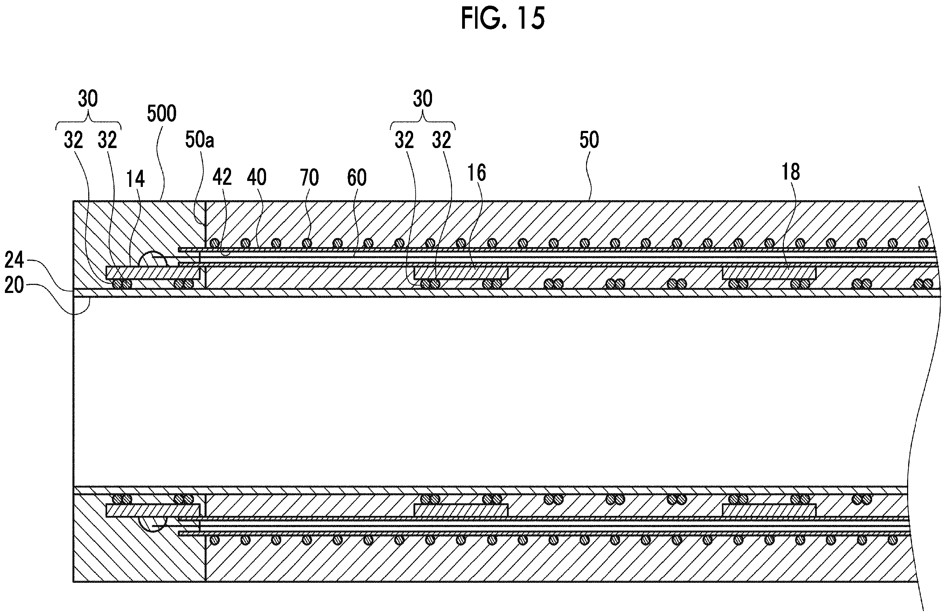

FIG. 15 is a longitudinal cross-sectional view illustrating a state where the outer layer 50 is formed again in a removed part of the outer layer 50 located on the distal side, which is added to the configuration illustrated in FIG. 14.

Firstly, an outline of the method for manufacturing the catheter 1 of the embodiment will be described.

The manufacturing method includes forming a reinforcing wire layer; fixing a marker; removing a reinforcing wire layer; and forming an outer layer.

Forming the reinforcing wire layer is a step in which the reinforcing wire layer 30 is formed by winding the reinforcing wire 32 on the inner layer 24 defining the main lumen 20.

Fixing the marker is a step in which each of the first marker 14 and the second marker 16 is fixed, each of which contains a radiopaque metallic material and has a ring shape, to the circumference of the reinforcing wire layer 30 by caulking such that the second marker 16 is positioned closer to the proximal side than the first marker 14.

Removing the reinforcing wire layer is a step in which the reinforcing wire layer 30 between the first marker 14 and the second marker 16 is removed.

Forming the outer layer is a step in which the outer layer 50 is formed so as to include the reinforcing wire layer 30, the first marker 14, and the second marker 16.

The manufacturing method further includes collective winding in which the holding wire 70 is wound collectively with the reinforcing wire layers 30, the first marker 14, and the second marker 16.

The manufacturing method further includes disposing a sub-tube in which the sub-tube 40 is disposed, so that the sub-tube defines the secondary lumen 42 having a smaller diameter than the main lumen 20, at a position outside the first marker 14 and the second marker 16 after removing the reinforcing wire layer; inserting an operating line in which the operating line 60 is movably inserted into the secondary lumen 42 after disposing the sub-tube; and connecting an operating line in which the tip end of the operating line 60 is connected to the first marker 14 after inserting the operating line.

Hereinbelow, the manufacturing method will be described in detail.

In the reinforcing wire layer forming step, firstly, the inner layer 24 is formed around a main core 22. The main core 22 is a mandrel (core), and is a wire rod having a circular cross section which defines the main lumen 20. The material of the main core 22 is not limited to a specific material, and a stainless steel is capable of being used as the material of the main core 22. It is possible to form the inner layer 24 by dipping the main core 22 in a coating solution obtained by dispersing a fluorinated polymer such as polytetrafluoroethylene (PTFE) in a solvent, and then by drying the main core 22 taken out of the coating solution.

Subsequently, as illustrated in FIG. 8, the reinforcing wire layer 30 is formed by braiding multiple turns of the reinforcing wire 32 in a mesh pattern on an outer surface of the inner layer 24.

Subsequently, in the marker fixing step, as illustrated in FIG. 9, the first marker 14 and the second marker 16 are fixed to the circumferences of the reinforcing wire layers 30 via caulking such that the second marker 16 is positioned closer to the proximal side than the first marker 14. The third marker 18 is also fixed, via caulking, to the circumference of the reinforcing wire layer 30 positioned closer to the proximal side than the second marker 16.

Subsequently, in the wire protective layer removing step, the reinforcing wire layer 30 between the first marker 14 and the second marker 16 is removed. The reinforcing wire layer 30 is capable of being removed by any means such as laser or a cutting blade.

Subsequently, in the sub-tube disposing step, the sub-tube 40, which defines the secondary lumen 42 having a smaller diameter than the main lumen 20, is disposed outside the first marker 14, the second marker 16, and the third marker 18.

The sub-tube 40 is formed around the secondary core 44 in advance. The secondary core 44 is a wire rod having a circular cross section which defines the secondary lumen 42. The material of the secondary core 44 is not limited to a specific material, and the same type of stainless steel as the main core 22 is capable of being used as the material of the secondary core 44. The secondary core 44 has a diameter thinner than the diameter of the main core 22. The sub-tube 40 preferably has a thickness thinner than the thickness of the inner layer 24. When the sub-tube 40 is prepared from a fluorinated polymer such as polytetrafluoroethylene (PTFE), it is possible to form the sub-tube 40 by dipping the secondary core 44 in a coating solution obtained by dispersing the polymer in a solvent, and then by drying the secondary core 44 taken out of the coating solution.

A cored tube may be prepared by drawing a material into a tube-shaped molded part while giving consideration to the requirement that the sub-tube 40 is to have an inner diameter larger than the outer diameter of the secondary core 44, and then by coating the circumference of the secondary core 44 with the molded part.

Subsequently, as illustrated in FIG. 12, in the collective winding step, the holding wire 70 collectively winds the reinforcing wire layers 30, the first marker 14, the second marker 16, and the sub-tube 40. In the manufacturing method, the sub-tube 40 is disposed above the first marker 14, the second marker 16, and the third marker 18 prior to the holding wire 70 being collectively wound therewith. The present disclosure is not limited to the sequence, and the disposition of the sub-tube 40 and the collective winding of the foregoing members with the holding wire 70 may be simultaneously performed.

Subsequently, as illustrated in FIG. 13, the outer layer is formed around the inner layer 24, the reinforcing wire layers 30, the first marker 14, the second marker 16, the secondary core 44, and the sub-tube 40. The outer layer 50 may be formed by coating extrusion in which a surface of a structure body is painted with a molten resin material. Alternatively, the outer layer 50 may be formed by mounting a resin ring or resin tube, which is preformed into an annular or tubular shape, around a structure body, and then by heat shaping the resultant structure body using a heat shrinkable tube.

In the subsequent operating line inserting step, firstly, a tip end part of the outer layer 50 is removed which is located at the distal portion DE of the tubular main body 10. The secondary core 44 is extended such that the diameter of the secondary core 44 is reduced, and thus the secondary core 44 with a reduced diameter separates from the sub-tube 40, and is withdrawn from the sub-tube 40. As a result, the secondary lumen 42 is formed in the sub-tube 40, and the operating line 60 is movably inserted into the secondary lumen 42.

In the operating line connecting step, the tip end of the operating line 60 is connected to an outer circumferential part of the first marker 14. The operating line is connected to the first marker 14 via soldering. A removed part 50a (located at the distal portion DE) of the outer layer 50 is formed to expose an outer circumferential portion of the first marker 14 to the outside for soldering.

Subsequently, as illustrated in FIG. 15, the removed part 50a of the outer layer 50 is backfilled. Similar to when the outer layer 50 is initially formed, a backfilled portion 500 may be formed by coating extrusion in which a surface of a structure body is painted with a molten resin material, or by mounting a resin ring or resin tube, which is preformed into an annular or tubular shape, around a structure body, and then by heat shaping the resultant structure body using a heat shrinkable tube.

Subsequently, the main lumen 20 is formed by withdrawing the main core 22 from the tubular main body 10.

In the manufacturing method, the tip end part of the tubular main body 10 is further shaped, specifically, is rounded by a method such as plastic deformation caused by grinding or heating (refer to FIG. 2). A hydrophilic layer (not illustrated) is formed on a surface of the outer layer, and the operating part 90 is attached to the base end portion of the tubular main body 10. As a result, it is possible to obtain the catheter 1 as a finished product.

In the present disclosure, the various configuration elements are not necessarily provided as individually independent members. A variety of combinations of configuration elements are allowed, for example, one member may be formed of a plurality of configuration elements, one configuration element may be formed of a plurality of members, a configuration element may be part of another configuration element, or part of a configuration element may be duplicated as part of another configuration element.

In the manufacturing method, a plurality of the steps are sequentially described; however, the sequence of the descriptions of the steps does not limit the sequence or timing to execute the plurality of steps. For this reason, when the manufacturing method is performed, the sequence of the plurality of steps is capable of being changed without departing from the content, and part or the entirety of the timings to execute the plurality of steps may be duplicated.

### Industrial Applicability

The present disclosure is capable of providing the catheter in which a reduction in diameter is capable of being realized without bendability or ease of assembly of the catheter being adversely affected, and the method for manufacturing the catheter.

### Reference Signs List

1: catheter
10: tubular main body
14: first marker
16: second marker
18: third marker
20: main lumen
22: main core
24: inner layer
30: reinforcing wire layer
32: reinforcing wire
40: sub-tube
42: secondary lumen
44: secondary core
50: outer layer
50a: removed part
60: operating line
70: holding wire
90: operating part
92: wheel operating part
94: main body case
95: recessed portion
96: hub
98: slider
500: backfilled portion
921: toothed engaging portion
DE: distal portion

## Claims

1. A catheter (1) comprising:
a reinforcing wire layer (30) wound on a main lumen (20);
a first marker (14) having a ring shape, and a second marker (16) having a ring shape and positioned closer to a proximal side than the first marker (14), each the first and second markers (14, 16) being provided around the reinforcing wire layer (30) and containing a radiopaque metallic material; and
an outer layer (50) including the reinforcing wire layer (30), the first marker (14), and the second marker (16),
wherein the reinforcing wire layer (30) is formed in a layer beneath the first marker (14) and the second marker (16),
wherein the catheter (1) further comprises a sub-tube, the sub-tube (40) defining a secondary lumen (42) having a smaller diameter than the main lumen (20); and
an operating line (60) which is movably inserted into the secondary lumen (42), and a tip end of which is connected to the first marker (14), and **characterized in that** the sub-tube (40) is disposed outside the first marker (14) and the second marker (16), and **in that** the region between the first marker (14) and the second marker (16) is a non-forming region in which the reinforcing wire layer (30) is not formed.

2. The catheter (1) according to claim 1, further comprising:
a holding wire (70) which collectively winds the reinforcing wire layer (30), the first marker (14), and the second marker (16),
wherein the first marker (14) and the second marker (16) are provided in a layer beneath the holding wire (70).

3. The catheter (1) according to claim 2,
wherein the holding wire (70) is a coil formed by winding a plurality of strands multiple turns.

4. The catheter (1) according to any one of claims 1 to 3,
wherein the reinforcing wire layer (30) is a mesh layer.

5. The catheter (1) according to claim 4,
wherein the reinforcing wire layer (30) is a plurality of mesh layer formed at the same layer level in front and back of the non-forming region.

6. The catheter (1) according to any one of claims 1 to 5,
wherein the first marker (14) has the same outer diameter as the second marker (16), and the first marker (14) and the second marker (16) are disposed at the same height in a radial direction of the catheter (1).

7. The catheter (1) according to any one of claims 1 to 6,
. wherein the outer layer (50) is formed of one type of material.

8. The catheter (1) according to any one of claims 1 to 7,
wherein the outer layer (50) has a substantially uniform thickness.

9. The catheter (1) according to any one of claims 2 to 8,
wherein a material of each of the reinforcing wire layer (30) and the holding wire (70) is a metallic material.

10. The catheter (1) according to any one of claims 1 to 9,
wherein a material of the sub-tube (40) is a thermoplastic polymer.

11. The catheter (1) according to any one of claims 1 to 10,
wherein the holding wire (70) is in contact with an outside surface of the sub-tube (40) at a position located opposite to an axis of the main lumen (20).

12. A method for manufacturing a catheter (1), the method comprising:
forming a reinforcing wire layer (30) in which a reinforcing wire layer (30) is formed by winding a reinforcing wire (32) on an inner layer (24) defining a main lumen (20);
fixing a marker in which each of a first marker (14) and a second marker (16) is fixed, each of which contains a radiopaque metallic material and has a ring shape, to a circumference of the reinforcing wire layer (30) by caulking such that the second marker is positioned closer to a proximal side than the first marker (14);
removing a reinforcing wire layer (30) in which the reinforcing wire layer between the first marker (14) and the second marker (16) is removed; and
forming an outer layer (50) in which an outer layer (50) is formed so as to include the reinforcing wire layer (30), the first marker (14), and the second marker (16).

13. The method for manufacturing a catheter (1) according to claim 12, further comprising:
collective winding in which a holding wire (70) is wound collectively with the reinforcing wire layer (30), the first marker (14), and the second marker (16) after removing the reinforcing wire layer (30).

14. The method for manufacturing a catheter (1) according to claim 12 or 13, further comprising:
disposing a sub-tube (40) in which a sub-tube (40) is disposed, so that the sub-tube (40) defines a secondary lumen (42) having a smaller diameter than the main lumen, at a position outside the first marker (14) and the second marker (16) after removing the reinforcing wire layer (30);
inserting an operating line (60) in which an operating line (60) is movably inserted into the secondary lumen (42) after disposing the sub-tube (40); and
connecting an operating line (60) in which a tip end of the operating line (60) is connected to the first marker (14) after inserting the operating line (60).

15. The method for manufacturing a catheter (1) according to any one of claims 12 to 14,
wherein the outer layer (50) is formed of one type of material.

16. The method for manufacturing a catheter (1) according to any one of claims 12 to 14,
wherein the outer layer (50) has a substantially uniform thickness.

17. The method for manufacturing a catheter (1) according to any one of claims 13 to 16,
wherein a material of each of the reinforcing wire layer (30) and the holding wire (70) is a metallic material.

18. The method for manufacturing a catheter (1) according to any one of claims 14 to 17,
wherein a material of the sub-tube (40) is a thermoplastic polymer.

19. The method according to any one of claims 14 to 18,
wherein the holding wire (70) is in contact with an outside surface of the sub-tube (40) at a position opposite to an axis of the main lumen (20).

## Patentansprüche

1. Katheter (1), umfassend:
eine Verstärkungsdrahtschicht (30), die um ein Hauptlumen (20) gewickelt ist;
eine erste Markierung (14), die eine Ringform aufweist, und eine zweite Markierung (16), die eine Ringform aufweist und näher an einer proximalen Seite als die erste Markierung (14) positioniert ist, wobei sowohl die erste als auch die zweite Markierung (14, 16) um die Verstärkungsdrahtschicht (30) herum vorgesehen sind und ein röntgendichtes metallisches Material enthalten; und
eine äußere Schicht (50), die die Verstärkungsdrahtschicht (30), die erste Markierung (14) und die zweite Markierung (16) enthält,
wobei die Verstärkungsdrahtschicht (30) in einer Schicht unterhalb der ersten Markierung (14) und der zweiten Markierung (16) ausgebildet ist,
wobei der Katheter (1) ferner ein Nebenrohr umfasst, wobei das Nebenrohr (40) ein sekundäres Lumen (42) definiert, das einen kleineren Durchmesser als das Hauptlumen (20) aufweist; und
eine Bedienleitung (60), die beweglich in das sekundäre Lumen (42) eingeführt ist und deren Spitzenende mit der ersten Markierung (14) verbunden ist, und
**dadurch gekennzeichnet, dass** das Nebenrohr (40) außerhalb der ersten Markierung (14) und der zweiten Markierung (16) angeordnet ist und dadurch, dass der Bereich zwischen der ersten Markierung (14) und der zweiten Markierung (16) ein nicht-formender Bereich ist, in dem die Verstärkungsdrahtschicht (30) nicht gebildet wird.

2. Katheter (1) nach Anspruch 1, ferner umfassend:
einen Haltedraht (70), der die Verstärkungsdrahtschicht (30), die erste Markierung (14) und die zweite Markierung (16) gemeinsam aufwickelt,
wobei die erste Markierung (14) und die zweite Markierung (16) in einer Schicht unter dem Haltedraht (70) vorgesehen sind.

3. Katheter (1) nach Anspruch 2, wobei der Haltedraht (70) eine Spule ist, die durch Wickeln einer Vielzahl von Litzen in mehreren Windungen gebildet wird.

4. Katheter (1) nach einem der Ansprüche 1 bis 3, wobei die Verstärkungsdrahtschicht (30) eine Netzschicht ist.

5. Katheter (1) nach Anspruch 4, wobei die Verstärkungsdrahtschicht (30) eine Vielzahl von Netzschichten ist, die auf dem gleichen Schichtniveau vor und hinter dem nicht-formenden Bereich ausgebildet sind.

6. Katheter (1) nach einem der Ansprüche 1 bis 5, wobei die erste Markierung (14) den gleichen Außendurchmesser wie die zweite Markierung (16) hat und die erste Markierung (14) und die zweite Markierung (16) in radialer Richtung des Katheters (1) auf der gleichen Höhe angeordnet sind.

7. Katheter (1) nach einem der Ansprüche 1 bis 6, wobei die äußere Schicht (50) aus einer Art von Material gebildet ist.

8. Katheter (1) nach einem der Ansprüche 1 bis 7, wobei die äußere Schicht (50) eine im Wesentlichen gleichmäßige Dicke aufweist.

9. Katheter (1) nach einem der Ansprüche 2 bis 8, wobei ein Material sowohl der Verstärkungsdrahtschicht (30) als auch des Haltedrahtes (70) ein metallisches Material ist.

10. Katheter (1) nach einem der Ansprüche 1 bis 9, wobei ein Material des Nebenrohrs (40) ein thermoplastisches Polymer ist.

11. Katheter (1) nach einem der Ansprüche 1 bis 10, wobei der Haltedraht (70) in Kontakt mit einer Außenfläche des Nebenrohrs (40) an einer Position ist, die einer Achse des Hauptlumens (20) gegenüberliegt.

12. Verfahren zur Herstellung eines Katheters (1), wobei das Verfahren umfasst:
Bilden einer Verstärkungsdrahtschicht (30), wobei eine Verstärkungsdrahtschicht (30) durch Wickeln eines Verstärkungsdrahtes (32) auf eine innere Schicht (24), die ein Hauptlumen (20) definiert, gebildet wird;
Befestigen einer Markierung, wobei sowohl eine erste Markierung (14) als auch eine zweite Markierung (16), von denen jede ein röntgendichtes metallisches Material enthält und eine Ringform aufweist, an einem Umfang der Verstärkungsdrahtschicht (30) durch Verstemmen befestigt ist, so dass die zweite Markierung näher an einer proximalen Seite als die erste Markierung (14) positioniert ist;
Entfernen einer Verstärkungsdrahtschicht (30), wobei die Verstärkungsdrahtschicht zwischen der ersten Markierung (14) und der zweiten Markierung (16) entfernt wird; und
Bilden einer äußeren Schicht (50), wobei eine äußere Schicht (50) so ausgebildet ist, dass sie die Verstärkungsdrahtschicht (30), die erste Markierung (14) und die zweite Markierung (16) enthält.

13. Verfahren zur Herstellung eines Katheters (1) nach Anspruch 12, ferner umfassend:
gemeinsames Wickeln, wobei ein Haltedraht (70) zusammen mit der Verstärkungsdrahtschicht (30), der ersten Markierung (14) und der zweiten Markierung (16) nach dem Entfernen der Verstärkungsdrahtschicht (30) gewickelt wird.

14. Verfahren zur Herstellung eines Katheters (1) nach Anspruch 12 oder 13, ferner umfassend:
Anordnen eines Nebenrohrs (40), wobei ein Nebenrohr (40) so an einer Position außerhalb der ersten Markierung (14) und der zweiten Markierung (16) nach dem Entfernen der Verstärkungsdrahtschicht (30) angeordnet wird, dass das Nebenrohr (40) ein sekundäres Lumen (42) mit einem kleineren Durchmesser als das Hauptlumen definiert,
Einführen einer Bedienleitung (60), wobei eine Bedienleitung (60) beweglich in das sekundäre Lumen (42) eingeführt wird, nachdem das Nebenrohr (40) angeordnet wurde; und
Verbinden einer Bedienleitung (60), wobei ein Spitzenende der Bedienleitung (60) nach dem Einführen der Bedienleitung (60) mit der ersten Markierung (14) verbunden wird.

15. Verfahren zur Herstellung eines Katheters (1) nach einem der Ansprüche 12 bis 14, wobei die äußere Schicht (50) aus einer Art von Material gebildet wird.

16. Verfahren zur Herstellung eines Katheters (1) nach einem der Ansprüche 12 bis 14, wobei die äußere Schicht (50) eine im Wesentlichen gleichmäßige Dicke aufweist.

17. Verfahren zur Herstellung eines Katheters (1) nach einem der Ansprüche 13 bis 16, wobei ein Material der Verstärkungsdrahtschicht (30) und des Haltedrahtes (70) jeweils ein metallisches Material ist.

18. Verfahren zur Herstellung eines Katheters (1) nach einem der Ansprüche 14 bis 17, wobei ein Material des Nebenrohrs (40) ein thermoplastisches Polymer ist.

19. Verfahren nach einem der Ansprüche 14 bis 18, wobei der Haltedraht (70) mit einer Außenfläche des Nebenrohrs (40) an einer Position gegenüber einer Achse des Hauptlumens (20) in Kontakt ist.

## Revendications

1. Cathéter (1) comportant :
une couche de fil de renfort (30) enroulée sur une lumière principale (20) ;
un premier marqueur (14) ayant une forme annulaire, et un second marqueur (16) ayant une forme annulaire et positionné plus près d'un côté proximal que le premier marqueur (14), chacun des premier et second marqueurs (14, 16) étant agencé autour de la couche de fil de renfort (30) et contenant un matériau métallique radio-opaque ; et
une couche extérieure (50) incluant la couche de fil de renfort (30), le premier marqueur (14) et le second marqueur (16),
dans lequel la couche de fil de renfort (30) est formée dans une couche située sous le premier marqueur (14) et le second marqueur (16),
dans lequel le cathéter (1) comporte en outre un sous-tube (40), le sous-tube (40) définissant une lumière secondaire (42) ayant un diamètre plus petit que la lumière principale (20) ; et
une ligne opératoire (60) qui est insérée dans la lumière secondaire (42) de manière mobile, et dont une extrémité d'embout est reliée au premier marqueur (14), et **caractérisé en ce que** le sous-tube (40) est disposé à l'extérieur du premier marqueur (14) et du second marqueur (16), et **en ce que** la zone située entre le premier marqueur (14) et le second marqueur (16) est une zone sans formation dans laquelle la couche de fil de renfort (30) n'est pas formée.

2. Cathéter (1) selon la revendication 1, comportant en outre :
un fil de retenue (70) qui enroule collectivement la couche de fil de renfort (30), le premier marqueur (14) et le second marqueur (16),
dans lequel le premier marqueur (14) et le second marqueur (16) sont agencés dans une couche située sous le fil de retenue (70).

3. Cathéter (1) selon la revendication 2,
dans lequel le fil de retenue (70) est une bobine formée en enroulant une pluralité de multiples spires de torons.

4. Cathéter (1) selon l'une quelconque des revendications 1 à 3,
dans lequel la couche de fil de renfort (30) est une couche de maillage.

5. Cathéter (1) selon la revendication 4,
dans lequel la couche de fil de renfort (30) est une pluralité de couches de maillage formées au même niveau de couche à l'avant et à l'arrière de la zone sans formation.

6. Cathéter (1) selon l'une quelconque des revendications 1 à 5,
dans lequel le premier marqueur (14) a le même diamètre extérieur que le second marqueur (16), et le premier marqueur (14) et le second marqueur (16) sont disposés à la même hauteur dans une direction radiale du cathéter (1).

7. Cathéter (1) selon l'une quelconque des revendications 1 à 6,
dans lequel la couche extérieure (50) est formée d'un type de matériau.

8. Cathéter (1) selon l'une quelconque des revendications 1 à 7,
dans lequel la couche extérieure (50) a une épaisseur sensiblement uniforme.

9. Cathéter (1) selon l'une quelconque des revendications 2 à 8,
dans lequel un matériau de chaque élément parmi la couche de fil de renfort (30) et le fil de retenue (70) est un matériau métallique.

10. Cathéter (1) selon l'une quelconque des revendications 1 à 9,
dans lequel un matériau du sous-tube (40) est un polymère thermoplastique.

11. Cathéter (1) selon l'une quelconque des revendications 1 à 10,
dans lequel le fil de retenue (70) est en contact avec une surface extérieure du sous-tube (40) à une position située à l'opposé d'un axe de la lumière principale (20).

12. Procédé de fabrication d'un cathéter (1), le procédé comportant :
la formation d'une couche de fil de renfort (30), une couche de fil de renfort (30) étant formée en enroulant un fil de renfort (32) sur une couche intérieure (24) définissant une lumière principale (20) ;
la fixation d'un marqueur, chaque marqueur parmi un premier marqueur (14) et un second marqueur (16) étant fixé, chacun contenant un matériau métallique radio-opaque et ayant une forme annulaire, à une circonférence de la couche de fil de renfort (30) par matage de telle sorte que le second marqueur est positionné plus près d'un côté proximal que le premier marqueur (14) ;
le retrait d'une couche de fil de renfort (30), la couche de fil de renfort entre le premier marqueur (14) et le second marqueur (16) étant retirée ; et
la formation d'une couche extérieure (50), une couche extérieure (50) étant formée de manière à inclure la couche de fil de renfort (30), le premier marqueur (14) et le second marqueur (16).

13. Procédé de fabrication d'un cathéter (1) selon la revendication 12, comportant en outre :
l'enroulement collectif, un fil de retenue (70) étant enroulé collectivement avec la couche de fil de renfort (30), le premier marqueur (14) et le second marqueur (16) après le retrait de la couche de fil de renfort (30).

14. Procédé de fabrication d'un cathéter (1) selon la revendication 12 ou 13, comportant en outre :
la mise en place d'un sous-tube (40), un sous-tube (40) étant disposé de sorte que le sous-tube (40) définit une lumière secondaire (42) ayant un diamètre plus petit que la lumière principale, à une position située à l'extérieur du premier marqueur (14) et du second marqueur (16) après retrait de la couche de fil de renfort (30) ;
l'insertion d'une ligne opératoire (60), une ligne opératoire (60) étant insérée dans la lumière secondaire (42) de manière mobile après la mise en place du sous-tube (40) ; et
le raccordement d'une ligne opératoire (60), une extrémité d'embout de la ligne opératoire (60) étant raccordée au premier marqueur (14) après l'insertion de la ligne opératoire (60).

15. Procédé de fabrication d'un cathéter (1) selon l'une quelconque des revendications 12 à 14,
dans lequel la couche extérieure (50) est formée d'un type de matériau.

16. Procédé de fabrication d'un cathéter (1) selon l'une quelconque des revendications 12 à 14,
dans lequel la couche extérieure (50) a une épaisseur sensiblement uniforme.

17. Procédé de fabrication d'un cathéter (1) selon l'une quelconque des revendications 13 à 16,
dans lequel un matériau de chaque élément parmi la couche de fil de renfort (30) et le fil de retenue (70) est un matériau métallique.

18. Procédé de fabrication d'un cathéter (1) selon l'une quelconque des revendications 14 à 17,
dans lequel un matériau du sous-tube (40) est un polymère thermoplastique.

19. Procédé selon l'une quelconque des revendications 14 à 18,
dans lequel le fil de retenue (70) est en contact avec une surface extérieure du tube secondaire (40) à une position opposée à un axe de la lumière principale (20).
